(19) Europäisches Patentamt
European Patent Office
Office européen des brevets

(11) Publication number: **0 505 937 A1**

(12) **EUROPEAN PATENT APPLICATION**

(21) Application number: **92104858.3**

(22) Date of filing: **20.03.92**

(51) Int. Cl.5: **C07H 17/07**, C07D 311/30, A61K 31/70, A61K 31/35

(30) Priority: **28.03.91 IT MI910846**

(43) Date of publication of application: **30.09.92 Bulletin 92/40**

(84) Designated Contracting States: **AT BE CH DE DK ES FR GB GR IT LI LU MC NL PT SE**

(71) Applicant: **GEYMONAT S.p.A.**
**2 Via S.Anna**
**I-03012 Anagni FR(IT)**

(72) Inventor: **Botre', Francesco**
**Via S. Anna, 2**
**I-03012 Anagni, (Frosinone)(IT)**
Inventor: **Conti, Ettore**
**Via S. Anna, 2**
**I-03012 Anagni, (Frosinone)(IT)**
Inventor: **Salvia, Giuseppe**
**Via S. Anna, 2**
**I-03012 Anagni, (Frosinone)(IT)**
Inventor: **Scibona, Giancarlo**
**Via S. Anna, 2**
**I-03012 Anagni, (Frosinone)(IT)**

(74) Representative: **Bianchetti, Giuseppe**
**Studio Consulenza Brevettuale Via Rossini, 8**
**I-20122 Milan(IT)**

(54) **Flavone derivatives, a process for the preparation thereof and pharmaceutical compositions containing them.**

(57) Flavone carbamoylated and sulfated derivatives, in form of salts with pharmaceutically acceptable metals or organic bases, have interesting antielastase activity.

EP 0 505 937 A1

The present invention relates to pharmaceutical compositions having antielastase activity , containing as the active ingredient one or more compounds of general formula (I):

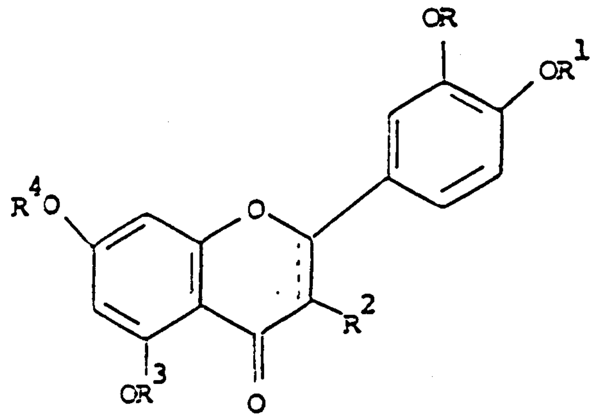

(I)

wherein
the bond at the 2-3 positions of the flavone ring can be single or double;
R is H, $CONR^5R^6$ or $SO_3X$;
$R^1$ is H, $CH_3$, $CONR^5R^6$ or $SO_3X$;
$R^2$ is H, or an O-disaccharide whose hydroxy hydrogens can be either partially or totally substituted by $CONR^5R^6$ and/or $SO_3X$ residues;
$R^3$ is H, $CONR^5R^6$ or $SO_3X$;
$R^4$ is H, $CONR^5R^6$, $SO_3X$ or a C-saccharide whose hydroxy hydrogens can be either partially or completely substituted by $CONR^5R^6$ and/or $SO_3X$ residues;
$R^5$ or $R^6$ are independently $C_1$-$C_4$ alkyl groups, or they form, together with the nitrogen atom they are linked to, a 5-7 membered ring, optionally containing another heteroatom, whereas
X is the equivalent of an alkali, alkaline-earth or earth metal, the ammonium cation or the cation of a pharmaceutically acceptable organic base;
with the proviso that at least a $CONR^5R^6$ residue and at least a $SO_3X$ are present.

Preferably, in the above formula $R^5$ and $R^6$ are alkyl $C_1$-$C_3$ residues (which can be the same or different) or the $NR_1R_2$ group is a pyrrolidino, piperidno or 4-methylpiperazino; whereas X is preferably sodium, potassium, magnesium, calcium, aluminium or the cation of a basic amino acid, for example lysine. Preferred compositions are those containing compounds (I) in which the $CONR^5R^6$ residue substitutes the hydrogen of one or two phenol hydroxy groups, and deriving from diosmin, esperidin, rutin, quercetin, quercitrin, diosmetin and esperetin.

Compounds of formula (I) deriving from diosmin were claimed in Italian Patent application n. 20829 A/90 in the Applicant's name. Said application also claims a process for the preparation thereof and evidences the better water-solubility and bioavailability compared with known diosmin derivatives as far as the antihemorrhagic and venotonic activities.

Now it has surprisingly been found that diosmin derivatives claimed in Italian patent application n. 20829 A/90 have interesting antielastase properties, which are also present in other flavones or flavonoids of general formula (I), which are here described and claimed.

Therefore, a further object of the present invention are compounds of formula (Ia):

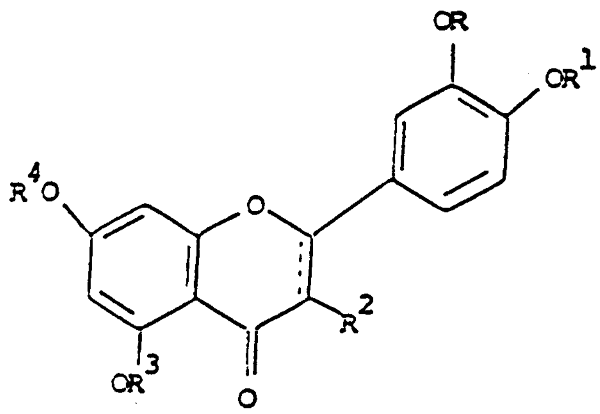

(Ia)

wherein
the bond at the 2-3 position of the flavone ring can be single or double;
R is H, $CONR^5R^6$ or $SO_3X$;
$R^1$ is H, $CH_3$, $CONR^5R^6$ or $SO_3X$;
$R^2$ is H, or a O-disaccharide whose hydroxy hydrogens can be partially or completely substituted by $CONR^5R^6$ and/or $SO_3X$ residues;
$R^3$ is H, $CONR^5R^6$ or $SO_3X$;
$R^4$ is H, $CONR^5R^6$, $SO_3X$ or a C-saccharide residue whose hydroxy hydrogens can be partially or completely substituted by $CONR^5R^6$ and/or $SO_3X$ residues;
$R^5$ or $R^6$ are independently $C_1$-$C_4$ alkyl groups, or they form, together with the nitrogen atom they are linked to, a 5-7 membered ring, optionally containing another heteroatom, whereas
X is the equivalent of an alkali, alkaline-earth or earth metal, the ammonium cation or the cation of a pharmaceutically acceptable organic base;
with the proviso that when a double bond is present between the 2 and 3 positions of the flavone ring, $R^4$ has meanings different from the rhamnose residue; and that in the molecule of formula (Ia) at least a $CONR^5R^6$ residue and at least a $SO_3X$ are present.

Compounds (Ia) can be prepared analogously to what described in the above cited Italian patent application n. 20829 A/90, by reacting flavones or flavonoids of formula (II):

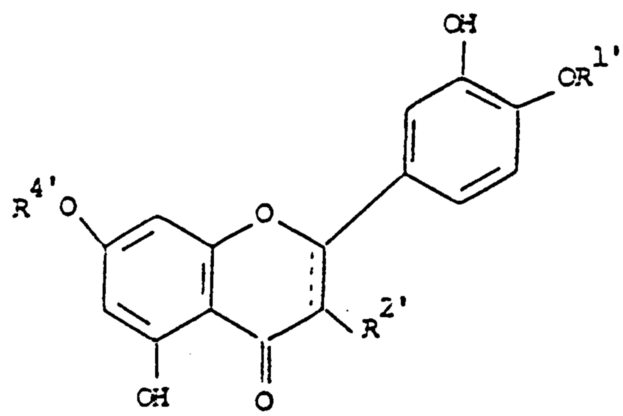

(II)

wherein the bond at the 2-3 position of the flavone ring can be single or double;
$R^{1'}$ is H or $CH_3$; whereas
$R^{2'}$ is H or an O-disaccharide residue;
$R^{4'}$ is H or a C-disaccharide residue;
with a carbamoyl chloride of formula (III):

$Cl$-$CO$-$NR^5R^6$    (III)

wherein $R^5$ and $R^6$ have the above mentioned meanings, to obtain an intermediate in which one or more

(but not all) hydroxy groups of the starting compound are substituted by $-O-CO-NR^5R^6$ groups (wherein $R^5$ and $R^6$ have the above mentioned meanings) then reacting said intermediate with the sulfuric anhydride-trimethylamine complex of formula (IV):

$SO_3.N(CH_3)_3$ (IV)

to obtain a sulfated compound in which at least a hydroxy group of compound (II) is substituted by an $O-SO_3^- N^+H(CH_3)_3$ group, the trimethylammoniun cation being optionally substituted, according to per se known methods, with other cations corresponding to the meaning of X, for example by reacting the above sulfated compound with an alkanoate of general formula (V)

$R^7COOX$ (V)

wherein X has the above mentioned meaning and $R^7$ is a $C_1$-$C_8$ straight or branched alkyl. Moreover it is possible to substitute, by means of traditional methods, any one of the X cations with another one.

The reaction sequence of the invention is carried out according to conventional techniques by reacting the compound (II) with the carbamoyl chloride (III) in an inert solvent, preferably aprotic polar, for example dimethylformamide or dimethylacetamide, preferably after salifying the hydroxy groups with a suitable base, for example an alkali hydride, preferably sodium hydride. The subsequent sulfation reaction can be carried out directly, without isolating the carbamoylated intermediate, in the same solvent as that used for the above step.

The sulfation is carried out preferably heating at mild temperature, generally from 50 to 80 °C, for some hours. The sulfated compound is recovered from the reaction mixture, but, if the trimethylammonium cation must be substituted by another cation, it is directly treated with alkanoate (V), without purification, to obtain the desired salt. The reaction is preferably carried out in an alkanol solvent such as ethanol or, even better, methanol, in which the final product is poorly soluble, directly precipitating in high purity.

Compounds (Ia) according to the invention are stable, have a satisfactory water-solubility and a high bioavailability. Pharmacological tests evidenced much higher antihemorrhagic and venotonic activities than those of the starting flavones or flavonoids, due to a better bioavailability.

Moreover compounds of formula (I) also have surprising antielastase characteristics. Therefore they are useful in the treatment of all those pathological conditions characterized by an abnormal elastase activity, which can be evidenced by degradation of connective tissue, such as in vasculopathies, lung emphysema, rheumatoid arthritis, circulatory diseases and in some inflammatory diseases.

Elastases are enzyme capable of degrading some important protein structures which are present in the organism and therefore are considered to be one of the main "destructive" enzymes present in human organism. They exert their action mainly on different kinds of collagene (I, II, III and IV), on proteoglycans and particularly on elastine. Elastases are located in leukocytes and therefore they can exert their action in very different districts in the organism and on very different substrates, such as immunoglobulins (G and M) and fibronectin.

For this reason, elastase have been considered responsible for a lot of pathological conditions characterized by a degradation of the connective tissue, such as lung emphysema, rheumatoid arthritis, coagulation disorders, inflammatory processes. More recently, various efforts have been carried out to prove elastases being involved in many vascular pathologies, particularly in venous insufficiency, and many hypotheses about the mechanism causing some pathologies have been formulated, one of the more trustworthy being that connects said pathologies with the etiology thereof.

In principle, the normal physiological condition is the result from a dynamic balance deriving from an appropriate interaction between elastase and antielastase activities. The factors responsible for such a dynamic balance are different, and any impairments of the action of one or more of said factors involves the onset of pathological conditions. Anyhow, the final effect can be considered an impairment of the elastin activity.

Therefore, synthetic or hemi-synthetic compounds having antielastase activity are highly required.

Such an activity was evidenced in various chemicals, such as peptidylchloromethylketones, peptide aldehydes, acyl and paranitrophenylcarbamates, alkylilisocianates and imidazolyl-N-carboxyamides, unsaturated fatty cis-acids, N-acylbenzoisothiazolinones and related 1,1-dioxides, as well as different beta-lactam derivatives of cefalosporins.

Reference is made to P. Lastienne et al., J. Biol. Chem.

**254**, 5219-5221 (1979); J. Powers et al., Biochemistry **12**, 4767-4774 (1973); R. Thompson, ibid. **12**, 47-51 (1973); H. Umezawa et al., J. Antibiotic. **26**, 787-789 (1973); C. Dorn et al., J. Med. Chem. **20**, 1464-1468 (1977); J.C. Powers et al., Biochem. Biophys. Res. Commun. **67**, 639-644 (1975); R.E. Schofield et al., Biochem **16**, 2492-2496 (1977); W.E. Brown et al., ibid. **12**, 828-840 (1973): W.C. Iroutas et al., Biochem. Biophys. Res. Commun. **95**, 1890-1894 (1980); B. Ashe et al., ibid. **75**, 194-199 (1977) and J. Biol. Chem. **256**,, 11603-11606 (1981); J.B. Doherty et al., Nature **322**,

192-194 (1986).

In view of what stated above, the antielastase activity of compounds (I) is even more surprising, in that the corresponding starting flavonoids (or flavones), and the derivatives thereof having carboxy or alcohol groups, or sulfuric groups when no carbamoyl residues are present, turned out to lack almost completely of such an activity.

The antielastase activity of compounds (I) was evidenced by the method described by Bieth and coll. (Biochim. med.

**11**,

350 (1974)), with slight changes when the test compounds were poorly water-soluble.

The determination of the antielastase activity was carried out using porcine pancreatic elastase (90 U/mg/weight; 120 U/mg protein-biuret-n.E. 0.127 Sigma) as the enzyme in solution. The enzyme-containing solution was prepared dissolving the enzyme in 0,2 M phosphate buffer, pH 8, to a final concentration of 1 microgram/ml.

The substrate solution was N-succinyl-L-alanyl-L-alanyl-L-alanyl-para-nitroanilide (available from Sigma cat. N.S. 4760). The substrate was dissolved in dimethylsulfoxide to a concentration of 5-6 mg/ml (125 mM).

The procedure to determine the antielastase activity consisted of the following steps:

- 2,9 ml of the enzyme solution with antielastase activity were placed into a quartz cuvette with 10 mm optical path, then 0,5 ml of bidistilled water were added. This standard solution was subsequently added with the different solutions of the various inhibitors.

The test solutions (standard and standard + inhibitor) were incubated at 30°C for 10', then 20 μl of substrate were added and the solution in the cuvette was stirred. The UV spectrum was recorded at 410 nm for 10' with a Beckman mod. 24 spectrophotometer against a blank consisting of the same enzyme-free standard solution.

The following Table summarizes the data from the test carried out on some samples.

TABLE

| INHIBITOR | $I_{50}$ (μg/ml) |
|---|---|
| Diosmin | - |
| Diosmin (with 2 sulfate groups)* | 181 |
| Diosmin (with 4 sulfate groups)* | 88 |
| Diosmin (with 1-3 sulfate groups)* | 236 |
| Diosmin (with 4-6 sulfate groups)* | 100 |
| Hydrosmin** | 623 |
| Esperidin | 570 |
| Compound of Ex. 2 | 132 |
| Rutin | 600 |
| Compound of Ex. 4 | 174 |

* and 2 dimethylcarbamoyl groups
** 3'-hydroxyethyldiosmin

It should be pointed out that no activity could be evidenced for diosmin as such, due to its low solubility in organic solvents. Nevertheless, the minimum concentration obtained (about 10 mg/ml) confirmed the absence of any antielastase activity.

The above data prove that the product obtained by carbamoylation and sulfation of the flavonoid is the one achieving favourable results as far as the antielastase activity is concerned; the higher the sulfation, the more evident the results.

The following examples illustrate the invention.

**EXAMPLE 1**

Example 1 of Italian patent application n. 20829 A/90 is reported as a reference. 60.85 mg (0.10

mmole) of diosmin are suspended in 10 ml of dimethylacetamide. 6 mg (0.25 mmole) of sodium hydride are added at room temperature and, after 3 hours, 26.9 mg (0.25 mmole) of N,N-dimethylcarbamoyl chloride are added.

At the end of the reaction (about 2 hours) 50 ml of dimethylacetamide and 208.65 mg (1.5 mmoles) of $(CH_3)_3N.SO_3$ are added to the reaction mixture, which is heated to 60-70 °C for 3 hours. Dimethylacetamide is distilled off under vacuum and the residue (trimethylammonium salt) is taken up into methanol. The methanol solution (optionally filtered on charcoal) is treated with a potassium acetate saturated solution in methanol, to precipitate the potassium salt which is washed with methanol and dried at 40 °C under vacuum. If the sodium salt is desired, the trimethylammonium salt is dissolved in methanol and treated with a saturated solution of sodium 2-ethylhexanoate in methanol.

| Elementary analysis for $C_{34}H_{36}N_2Na_6O_{35}S_6$ | | |
|---|---|---|
| | calculated % | found % |
| C | 29.96 | 30.03 |
| H | 2.66 | 2.74 |
| N | 2.05 | 2.01 |

## EXAMPLE 2

61.06 mg (0.10 mmole) of esperidin suspended in 10 ml of dimethylacetamide are added with 6.00 mg (0.25 mmole) of NaH, at room temperature. After 3 hour stirring, 33.40 mg (0.25 mmole) of N,N-dimethylcarbamoyl chloride are added thereto.

After two more hours, 40 ml of dimethylacetamide and 208.5 mg (1.5 mmoles) of $(CH_3)_3N.SO_3$ complex are added.

The procedure of Example 1 is followed, to obtain the desired sodium salt.

| Elementary analysis for $C_{34}H_{38}Na_6N_2O_{35}S_6$ | | |
|---|---|---|
| | calculated % | found % |
| C | 29.89 | 29.78 |
| H | 2.72 | 2.80 |
| N | 2.01 | 1.96 |

## EXAMPLE 3

Following the procedure of Example 1, starting from esperidin and 4-methyl-piperazin-1-yl-carbamoyl chloride, a corresponding derivative is obtained, having two carbamoyl residues and 6 sulfuric residues.

| Elementary analysis for $C_{40}H_{44}Na_6N_4O_{35}S_6$ | | |
|---|---|---|
| | calculated % | found % |
| C | 32.60 | 32.73 |
| H | 2.96 | 3.02 |
| N | 3.75 | 3.71 |

## EXAMPLE 4

A suspension of 61.05 mg (0.1 mmole) of rutin is treated, following the procedure of Example 1, with N,N-dimethylcarbamoyl chloride and then with the $(CH_3)_3N.SO_3$ complex, to obtain a potassium salt of formula $C_{33}H_{34}K_6N_2O_{36}S_6$, which formula is confirmed by the elementary analysis.

**EXAMPLE 5**

302.2 mg (1 mmole) of quercetin are suspended in the cold in 30 ml of dimethylformamide; 60 mg (2.5 mmoles) of NaH and, after three hour stirring at room temperature, 270 mg (2.5 mmoles) of N,N-dimethylcarbamoyl chloride are added thereto. After three more hours, the reaction mixture is diluted with 80 ml more of dimethylformamide and 278 mg (3 mmoles) of $(CH_3)_3N.SO_3$. The procedure of Example 1 is followed, to isolate the disodium salt of formula $C_{21}H_{18}N_3Na_2O_{15}S_2$, which is confirmed by the elementary analysis.

**EXAMPLES 6-10**

Following the procedures described in the above examples, the following compounds of formula (I) were prepared:

- from rutin: di-(pyrrolidylcarbamoyl-derivative)-hexasulfate, hexasodium salt;
- from rutin: di-(piperidylcarbamoyl-derivative)-tetrasulfate, tetralysine salt;
- from esperidin: tri-(dimethylcarbamoyl-derivative)-disulfate, dipotassium salt;
- from esperetin: mono-pyrrolidylcarbamoyl-derivative, monosulfate, sodium salt;
- from diosmin: di-(dimethylcarbamoyl-derivative), disulfate, dilysine salt.

## Claims

1. Pharmaceutical compositions having antielastase activity, containing as the active ingredient one or more flavone and flavonoid derivatives of general formula (I):

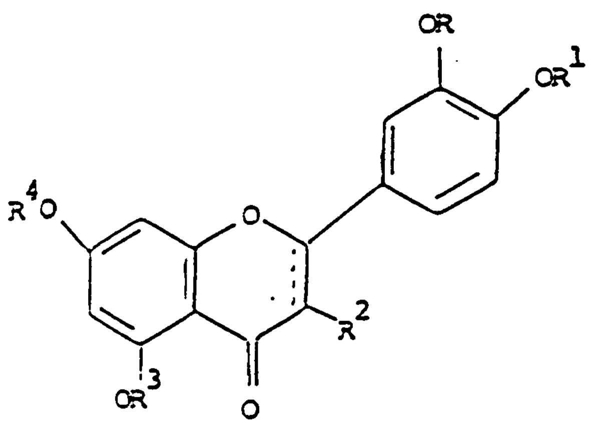

(I)

wherein
the bond at the 2-3 positions of the flavone ring can be single or double;
R is H, $CONR^5R^6$ or $SO_3X$;
$R^1$ is H, $CH_3$, $CONR^5R^6$ or $SO_3X$;
$R^2$ is H, or an O-disaccharide whose hydroxy hydrogens can be either partially or totally substituted by $CONR^5R^6$ and/or $SO_3X$ residues;
$R^3$ is H, $CONR^5R^6$ or $SO_3X$;
$R^4$ is H, $CONR^5R^6$, $SO_3X$ or a C-saccharide whose hydroxy hydrogens can be either partially or completely substituted by $CONR^5R^6$ and/or $SO_3X$ residues;
$R^5$ or $R^6$ are independently $C_1$-$C_4$ alkyl groups, or they form, together with the nitrogen atom they are linked to, a 5-7 membered ring, optionally containing another heteroatom, whereas
X is the equivalent of an alkali, alkaline-earth or earth metal, the ammonium cation or the cation of a pharmaceutically acceptable organic base;
with the proviso that at least a $CONR^5R^6$ residue and at least a $SO_3X$ are present.

2. Compounds of general formula (Ia):

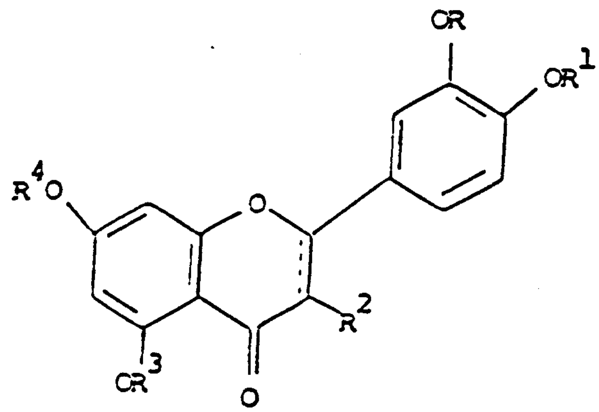

(Ia)

wherein

the bond at the 2-3 positions of the flavone ring can be single or double;

R is H, $CONR^5R^6$ or $SO_3X$;

$R^1$ is H, $CH_3$, $CONR^5R^6$ or $SO_3X$;

$R^2$ is H, or an O-disaccharide whose hydroxy hydrogens can be either partially or totally substituted by $CONR^5R^6$ and/or $SO_3X$ residues;

$R^3$ is H, $CONR^5R^6$ or $SO_3X$;

$R^4$ is H, $CONR^5R^6$, $SO_3X$ or a C-saccharide whose hydroxy hydrogens can be either partially or completely substituted by $CONR^5R^6$ and/or $SO_3X$ residues;

$R^5$ or $R^6$ are independently $C_1$-$C_4$ alkyl groups, or they form, together with the nitrogen atom they are linked to, a 5-7 membered ring, optionally containing another heteroatom, whereas

X is the equivalent of an alkali, alkaline-earth or earth metal, the ammonium cation or the cation of a pharmaceutically acceptable organic base;

with the proviso that when a double bond is present between the 2 and 3 positions of the flavone ring, $R^4$ has meanings different from the rhamnose residue, and that in the molecule of formula (Ia) at least a $CONR^5R^6$ residue and at least a $SO_3X$ are present.

3. Compounds according to claim 2, characterized in that $R^5$ and $R^6$ are $C_1$-$C_3$ alkyl groups, which can be the same or different.

4. Compounds according to claim 2, characterized in that the $NR^5R^6$ group is a pyrrolidino, piperidino or 4-methylpiperazino residue.

5. Compounds according to claims 2-4, characterized in that X is sodium, potassium, magnesium, calcium, aluminium or the cation of a basic amino acid.

6. Compounds according to claims 2-5, characterized in that the $CONR^5R^6$ residue substitutes the hydrogen of one or two phenol hydroxy groups.

7. Compounds according to claims 1-5, deriving from esperidin, rutin, quercetin, quercitrin, diosmetin and esperetin.

8. A process for the preparation of the compounds according to claims 2-7, characterized in that flavones or flavonoids of formula (II)

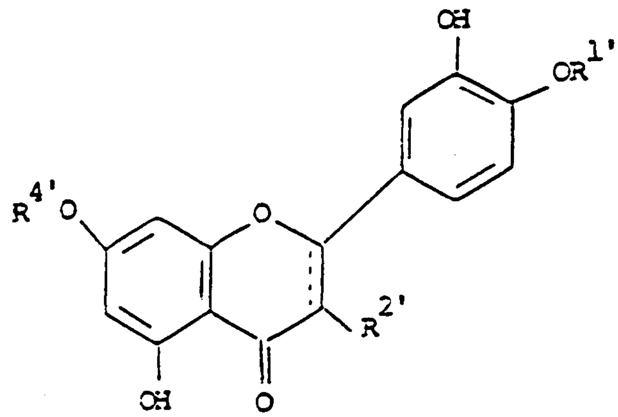

(II)

wherein
the bond at the 2-3 positions of the flavone ring can be single or double;
$R^{1'}$ is H or $CH_3$; whereas
$R^{2'}$ is H or an O-disaccharide residue;
$R^{4'}$ is H or a C-disaccharide residue;
are reacted with a carbamoyl chloride of formula (III):

$Cl\text{-}CO\text{-}NR^5R^6$ (III)

wherein $R^5$ and $R^6$ have the above defined meanings, in that the resulting intermediate containing one or more carbamoyl groups is sulfated with the sulfuric anhydride-trimethylamine complex of formula (IV):

$SO_3.N(CH_3)_3$ (IV)

and in that in the resulting sulfated compound, containing one or more $O\text{-}SO_3NH(CH_3)_3$ groups, the trimethylammonium cation is optionally substituted, according to per se known methods, with other cations corresponding to the meaning of X.

**9.** A process according to claim 8, characterized in that the reaction of II and III is carried out in a polar aprotic solvent.

**10.** A process according to claims 8 and 9, characterized in that the sulfation reaction is carried out without isolating the carbamoylated intermediate, directly in the same solvent in which it has been obtained, by heating at 50-80 ° C.

**11.** Pharmaceutical compositions containing one or more compounds of claims 1 and 2 together with pharmaceutically acceptable carriers and excipients.

**Claims for the following Contracting States : ES, GR, PT**

**1.** A process for the preparation of compounds of general formula (Ia):

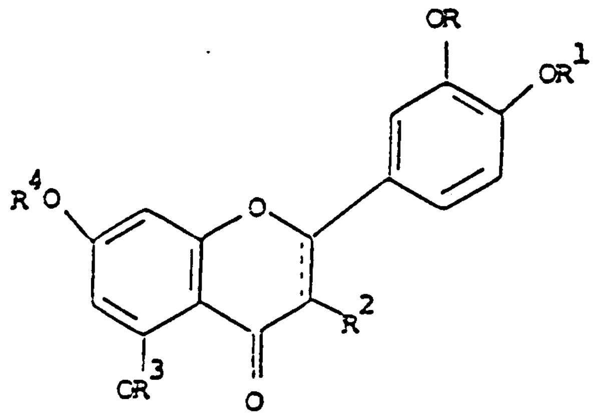

(Ia)

wherein
the bond at the 2-3 positions of the flavone ring can be single or double;
R is H, $CONR^5R^6$ or $SO_3X$;
$R^1$ is H, $CH_3$, $CONR^5R^6$ or $SO_3X$;
$R^2$ is H, or an O-disaccharide whose hydroxy hydrogens can be either partially or totally substituted by $CONR^5R^6$ and/or $SO_3X$ residues;
$R^3$ is H, $CONR^5R^6$ or $SO_3X$;
$R^4$ is H, $CONR^5R^6$, $SO_3X$ or a C-saccharide whose hydroxy hydrogens can be either partially or completely substituted by $CONR^5R^6$ and/or $SO_3X$ residues;
$R^5$ or $R^6$ are independently $C_1$-$C_4$ alkyl groups, or they form, together with the nitrogen atom they are linked to, a 5-7 membered ring, optionally containing another heteroatom, whereas
X is the equivalent of an alkali, alkaline-earth or earth metal, the ammonium cation or the cation of a pharmaceutically acceptable organic base;
with the proviso that when a double bond is present between the 2 and 3 positions of the flavone ring, $R^4$ has meanings different from the rhamnose residue; and that in the molecule of formula (Ia) at least a $CONR^5R^6$ residue and at least a $SO_3X$ are present,
characterized in that flavones or flavonoids of formula (II)

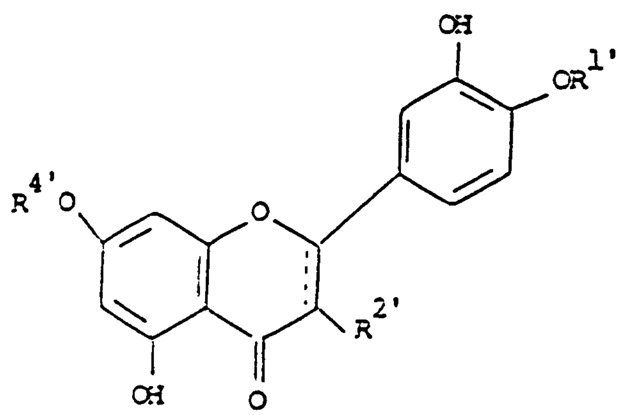

(II)

wherein
the bond at the 2-3 positions of the flavone ring can be single or double;
$R^{1'}$ is H or $CH_3$; whereas
$R^{2'}$ is H or an O-disaccharide residue;
$R^{4'}$ is H or a C-disaccharide residue;
are reacted with a carbamoyl chloride of formula (III):

$Cl-CO-NR^5R^6$ (III)

wherein $R^5$ and $R^6$ have the above defined meanings, in that the resulting intermediate containing one

or more carbamoyl groups is sulfated with the sulfuric anhydride-trimethylamine complex of formula (IV):

$SO_3.N(CH_3)_3$ (IV)

and in that in the resulting sulfated compound, containing one or more $O\text{-}SO_3NH(CH_3)_3$ groups, the trimethylammonium cation is optionally substituted, according to per se known methods, with other cations corresponding to the meaning of X.

**2.** A process according to claim 1, characterized in that the reaction of II and III is carried out in a polar aprotic solvent.

**3.** A process according to claims 1 and 2, characterized in that the sulfation reaction is carried out without isolating the carbamoylated intermediate, directly in the same solvent in which it has been obtained, by heating at 50-80 °C.

European Patent Office

# EUROPEAN SEARCH REPORT

Application Number

EP 92 10 4858

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 013 470 (AMERICAN CYANAMID)<br>* pages 5-6 *<br>--- | 2,11 | C07H17/07<br>C07D311/30<br>A61K31/70<br>A61K31/35 |
| A | US-A-4 202 825 (M.M. TAYA et al.)<br>* column 1 *<br>----- | 2,11 | |
| | | | TECHNICAL FIELDS SEARCHED (Int. Cl.5) |
| | | | C07H<br>C07D |

The present search report has been drawn up for all claims

| Place of search | Date of completion of the search | Examiner |
|---|---|---|
| THE HAGUE | 27 MAY 1992 | RUSSELL F. ENGLISH |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)